(19) Europäisches Patentamt
European Patent Office
Office européen des brevets

(11) EP 0 688 315 B1

(12) EUROPÄISCHE PATENTSCHRIFT

(45) Veröffentlichungstag und Bekanntmachung des Hinweises auf die Patenterteilung:
**06.05.1999 Patentblatt 1999/18**

(21) Anmeldenummer: **94908350.5**

(22) Anmeldetag: **19.02.1994**

(51) Int Cl.[6]: **C07D 207/40**, A61K 31/40, C07D 401/12, C07D 403/12, A61K 31/445

(86) Internationale Anmeldenummer:
**PCT/EP94/00481**

(87) Internationale Veröffentlichungsnummer:
**WO 94/21607 (29.09.1994 Gazette 1994/22)**

# (54) PYRROLIDIN- UND OXAZOLIDIN DERIVATE , IHRE HERSTELLUNG UND IHRE VERWENDUNG ALS THROMBOCYTEN-AGGREGATIONS-HEMMER

PYRROLIDINE AND OXAZOLIDINE DERIVATIVES, THEIR PREPARATION AND THEIR USE AS INHIBITORS OF THROMBOCYTE AGGREGATION

DERIVES DE LA PYRROLIDINE OU DE L' OXAZOLIDINE , LEUR PREPARATION ET LEUR UTILISATION COMME INHIBITEURS DE L'AGGREGATION DE THROMBOCYTES

(84) Benannte Vertragsstaaten:
**AT BE CH DE DK ES FR GB GR IE IT LI LU MC NL PT SE**

(30) Priorität: **13.03.1993 DE 4308034**

(43) Veröffentlichungstag der Anmeldung:
**27.12.1995 Patentblatt 1995/52**

(73) Patentinhaber: **HOECHST AKTIENGESELLSCHAFT**
**65929 Frankfurt am Main (DE)**

(72) Erfinder:
- **KLINGLER, Otmar**
**D-63110 Rodgau (DE)**
- **BREIPOHL, Gerhard**
**D-60529 Frankfurt am Main (DE)**
- **ZOLLER, Gerhard**
**D-61137 Schöneck (DE)**
- **JABLONKA, Bernd**
**D-65812 Bad Soden (DE)**
- **JUST, Melitta**
**D-63225 Langen (DE)**
- **KNOLLE, Jochen**
**D-65830 Kriftel (DE)**
- **KÖNIG, Wolfgang**
**D-94375 Stallwang (DE)**

(56) Entgegenhaltungen:
**EP-A- 0 126 849**
**EP-A- 0 190 755**
**EP-A- 0 449 079**
**EP-A- 0 512 831**
**EP-A- 0 530 505**
**GB-A- 1 539 817**
**GB-A- 2 032 419**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 688 315 B1

## Beschreibung

Die vorliegende Erfindung betrifft substituierte Heterocyclen, ihre Herstellung und ihre Verwendung als Heilmittel, insbesondere als Hemmstoffe der Blutplättchenaggregation.

In der EP-A 449 079 und der EP-A 530 505 sind Hydantoinderivate beschrieben, die thrombozytenaggregationshemmende Wirkungen aufweisen. Die EP-A 512 831 beschreibt Verbindungen, die die Fibrinogenbindung an Blutplättchen und dadurch die Aggregation der Plättchen verhindern. Weitere Untersuchungen zeigten, daß auch die Verbindungen der vorliegenden Erfindung starke Hemmstoffe der Blutplättchenaggregation sind.

Gegenstand der vorliegenden Erfindung sind neue Heterocyclen der allgemeinen Formel I

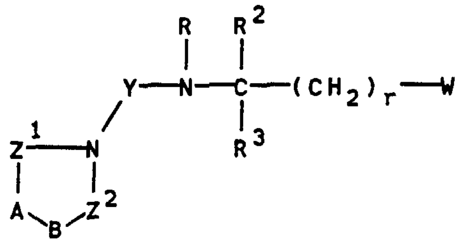

(I)

worin

$Z^1$

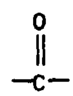 oder 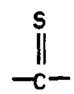

bedeutet;

$Z^2$

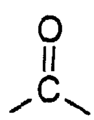 oder 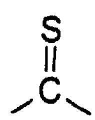

bedeutet;

Y -$(CH_2)_m$-CO-, wobei m für eine ganze Zahl von 1 bis 4 steht, oder -$CHR^s$-CO-oder

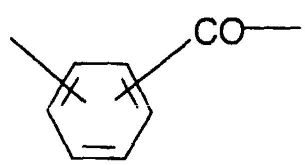

bedeutet;

r eine ganze Zahl von 0 bis 3 bedeutet;

A -$CHR^1$- bedeutet;

B -$CH_2$- oder -O- bedeutet;

W -$COW^1$, Tetrazolyl, -$SO_2$-OH oder -$SO_2$-$NHR^9$ bedeutet;

$W^1$ Hydroxy, ($C_1$-$C_{28}$)-Alkoxy, ($C_6$-$C_{14}$)-Aryl-($C_1$-$C_8$)-alkoxy, ($C_6$-$C_{14}$)-Aryloxy, Amino oder Mono- oder Di-(($C_1$-$C_{18}$)-Alkyl)-amino bedeutet;

R Wasserstoff oder ($C_1$-$C_6$)-Alkyl bedeutet;

$R^1$

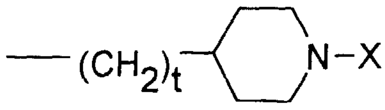

oder $-(CH_2)_n$-NH-X oder $-(CH_2)_p-C_6H_4-(CH_2)_q$-NH-X oder $-(CH_2)_p$-C(=NX)-$NH_2$ oder $-(CH_2)_p-C_6H_4-(CH_2)_q$-C(=NX)-$NH_2$ bedeutet, worin n für eine Zahl von 1 bis 6 steht, p und q unabhängig voneinander für eine Zahl von 0 bis 3 stehen und t für eine Zahl von 0 bis 2 steht;

X Wasserstoff, $(C_1-C_6)$-Alkyl, $(C_1-C_6)$-Alkylcarbonyl, $(C_1-C_6)$-Alkoxycarbonyl $(C_1-C_{18})$-Alkylcarbonyloxy-$(C_1-C_6)$-alkoxycarbonyl, $(C_6-C_{14})$-Arylcarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy oder Amino oder einen Rest der Formel II

R'-NH-C(=N-R")- (II)

bedeutet, wobei R' und R" unabhängig voneinander für Wasserstoff, $(C_1-C_6)$-Alkyl, Trifluor-$(C_1-C_6)$-alkyl, $(C_1-C_6)$-Alkoxycarbonyl, $(C_1-C_6)$-Alkylcarbonyl, $(C_6-C_{14})$-Arylcarbonyl, $(C_1-C_{18})$-Alkylcarbonyloxy-$(C_1-C_6)$-alkoxycarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy oder Amino stehen;

$R^2$ Wasserstoff, $(C_1-C_4)$-Alkyl oder Phenyl bedeutet, wobei das $(C_1-C_4)$-Alkyl und das Phenyl unsubstituiert oder ein- oder mehrfach mit gleichen oder verschiedenen Resten aus der Reihe Hydroxy, Amino, $(C_1-C_4)$-Alkoxy, Imidazolyl, Indolyl, Pyrrolidinyl, Hydroxypyrrolidinyl, Phenyl oder Halogen substituiert sein kann;

$R^3$ Wasserstoff, $-COOR^4$, $-CO-N(CH_3)-R^4$ oder $-CO-NH-R^4$ bedeutet;

$R^4$ Wasserstoff oder $(C_1-C_{28})$-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-($(C_1-C_{18})$-alkyl)-aminocarbonyl, Amino-$(C_2-C_{18})$-alkylaminocarbonyl, Amino-$(C_1-C_3)$-alkyl-phenyl-$(C_1-C_3)$-alkylaminocarbonyl, $(C_1-C_{18})$-Alkylcarbonylamino-$(C_1-C_3)$-alkylphenyl-$(C_1-C_3)$-alkylaminocarbonyl, $(C_1-C_{18})$-Alkylcarbonyl-amino-$(C_2-C_8)$-alkylaminocarbonyl, $(C_6-C_{14})$-Aryl-$(C_1-C_8)$-alkoxycarbonyl, Amino, Mercapto, $(C_1-C_{18})$-Alkoxy, $(C_1-C_{18})$-Alkoxycarbonyl, $(C_3-C_8)$-Cycloalkyl, Halogen, Nitro, Trifluormethyl oder durch den Rest $R^5$ substituiert sein kann, wobei

$R^5$ $(C_6-C_{14})$-Aryl, $(C_6-C_{14})$-Aryl-$(C_1-C_8)$-alkyl, einen mono- oder bizyklischen 5-bis 12-gliedrigen heterocyclischen Ring, der aromatisch, teilhydriert oder vollständig hydriert sein kann und der ein, zwei oder drei gleiche oder verschiedene Heteratome aus der Reihe Stickstoff, Sauerstoff und Schwefel enthalten kann, einen Rest $R^6$ oder einen Rest $R^6$CO- bedeutet, wobei der Aryl- und unabhängig davon der Heterozyklus-Rest ein- oder mehrfach durch gleiche oder verschiedene Reste aus der Reihe $(C_1-C_{18})$-Alkyl, $(C_1-C_{18})$-Alkoxy, Halogen, Nitro, Amino oder Trifluormethyl substituiert sein können;

$R^6$ $-NR^7R^8$, $-OR^7$, $-SR^7$, $-SO_2$-OH, $-SO_2NHR^9$, Tetrazolyl, eine Aminosäureseitenkette, einen natürlichen oder unnatürlichen Aminosäure-, Iminosäure-, Azaaminosäure- oder einen Dipeptid-Rest sowie deren Ester und Amide bedeutet, wobei freie funktionelle Gruppen durch in der Peptidchemie übliche Schutzgruppen geschützt sein können;

$R^7$ Wasserstoff, $(C_1-C_{18})$-Alkyl, $(C_6-C_{14})$-Aryl-$(C_1-C_8)$-alkyl, $(C_1-C_{18})$-Alkyl-carbonyl, $(C_1-C_{18})$-Alkoxycarbonyl,$(C_6-C_{14})$-Arylcarbonyl, $(C_6-C_{14})$-Aryl-$(C_1-C_8)$-alkylcarbonyl oder $(C_6-C_{14})$-Aryl-$(C_1-C_{18})$-alkyloxycarbonyl wobei die Alkylgruppen gegebenenfalls durch eine Aminogruppe substituiert und/oder wobei die Arylreste ein- oder mehrfach; vorzugsweise einfach, durch gleiche oder verschiedene Reste aus der Reihe $(C_1-C_8)$-Alkyl, $(C_1-C_8)$-Alkoxy, Halogen, Nitro, Amino und Trifluormethyl substituiert sein können, einen natürlichen oder unnatürlichen Aminosäure-, Iminosäure-, Azaaminosäure- oder einen Dipeptid-Rest bedeutet;

$R^8$ Wasserstoff, $(C_1-C_{18})$-Alkyl,$(C_6-C_{14})$-Aryl oder $(C_6-C_{14})$-Aryl-$(C_1-C_8)$-alkyl, bedeutet;

$R^9$ Wasserstoff, Aminocarbonyl, $(C_1-C_{18})$-Alkylaminocarbonyl, $(C_3-C_8)$-Cycloalkylaminocarbonyl, $(C_1-C_{18})$-Alkyl oder $(C_3-C_8)$-Cycloalkyl bedeutet;

$R^s$ eine Aminosäureseitenkette bedeutet;

sowie deren physiologisch verträgliche Salze;
wobei sich Aminosäurereste bzw. Aminosäureseitenketten von folgenden Aminosäuren ableiten können:
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp,

hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Cycloalkylreste sind insbesondere Cyclopropyl, Cyclobutyl, Cyclopentyl, Cyclohexyl, Cycloheptyl und Cyclooctyl, die aber auch durch beispielsweise ($C_1$-$C_4$)-Alkyl substituiert sein können. Beispiele für substituierte Cycloalkylreste sind 4-Methylcyclohexyl und 2,3-Dimethylcyclopentyl.

Alkylreste können geradkettig oder verzweigt sein. Dies gilt auch, wenn sie Substituenten tragen oder als Substituenten anderer Reste auftreten. Beispiele für geeignete $C_1$-$C_{28}$-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Nonadecyl, Eicosyl, Docosyl, Tricosyl, Pentacosyl, Hexacosyl, Heptacosyl, Octacosyl, Isopropyl, Isopentyl, Neopentyl, Isohexyl, 3-Methylpentyl, 2,3,5-Trimethylhexyl, sec.-Butyl, tert.-Butyl, tert.-Pentyl. Bevorzugte Alkylreste sind Methyl, Ethyl, Propyl, Isopropyl, Butyl, Isobutyl, sec.-Butyl und tert.-Butyl.

($C_6$-$C_{14}$)-Arylgruppen sind beispielsweise Phenyl, Naphthyl, Biphenylyl oder Fluorenyl, wobei Phenyl und Naphthyl bevorzugt sind. Entsprechendes gilt für Reste wie Aralkyl oder Arylcarbonyl. Aralkylreste sind insbesondere Benzyl sowie 1- und 2-Naphthylmethyl, die auch substituiert sein können. Substituierte Aralkylreste sind beispielsweise Halobenzyl oder ($C_1$-$C_4$)-Alkoxybenzyl.

Ist Phenyl zweifach substituiert, können die Substituenten in 1,2-, 1,3- oder 1,4-Position zueinander stehen. Die 1,3- sowie die 1,4-Position sind bevorzugt.

Mono- oder bicyclische 5- bis 12-gliedrige heterocyclische Ringe sind beispielsweise Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl oder ein benzanelliertes, cyclopenta-, cyclohexa- oder cyclohepta-anelliertes Derivat dieser Reste.

Diese Heterocyclen können an einem Stickstoffatom durch ($C_1$-$C_7$)-Alkyl, z. B. Methyl oder Ethyl, Phenyl oder Phenyl-($C_1$-$C_4$)-alkyl, z. B. Benzyl und/oder an einem oder mehreren Kohlenstoffatomen durch ($C_1$-$C_4$)-Alkyl, Halogen, Hydroxy, ($C_1$-$C_4$)-Alkoxy, z. B. Methoxy, Phenyl-($C_1$-$C_4$)-alkoxy, z. B. Benzyloxy, oder Oxo substituiert und aromatisch oder teilweise oder vollständig gesättigt sein. Stickstoffheterocyclen können auch als N-Oxide vorliegen.

Derartige Reste sind beispielsweise 2- oder 3-Pyrrolyl, Phenyl-pyrrolyl, z. B. 4- oder 5-Phenyl-2-pyrrolyl, 2-Furyl, 2-Thienyl, 4-Imidazolyl, Methyl-imidazolyl, z. B. 1-Methyl-2-, 4- oder 5-imidazolyl, 1,3-Thiazol-2-yl, 2-, 3- oder 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, z. B. 1-Methyl-, 5-Methyl-, 5-Methoxy, 5-Benzyloxy, 5-Chlor oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3-oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl. Teilhydrierte oder vollständig hydrierte heterocyclische Ringe sind beispielsweise Dihydropyridinyl, Pyrrolidinyl, z. B. 2-, 3- oder 4-(N-methylpyrrolidinyl), Piperazinyl, Morpholinyl, Thiomorpholinyl, Tetrahydrothienyl, Benzodioxolanyl.

Halogen steht für Fluor, Chlor, Brom oder Jod, insbesondere für Fluor oder Chlor.

Natürliche und unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band XV/1 und 2, Stuttgart, 1974):

Aad, Abu γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp,

Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Unter Aminosäureseitenketten werden Seitenketten von natürlichen oder unnatürlichen Aminosäuren verstanden. Azaaminosäuren sind natürliche oder unnatürliche Aminosäuren, in denen der Zentralbaustein

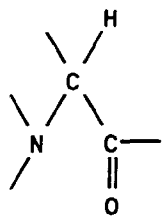 durch 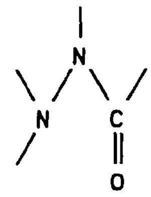

ersetzt ist.

Als Rest einer Iminosäure kommen insbesondere Reste von Heterocyclen aus der folgenden Gruppe in Betracht:

Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo [3.1.0]-hexan-3-carbonsäure; 2-Azaspiro[4.4]nonan-3-carbonsäure; 2-Azaspiro[4.5]decan-3-carbonsäure; Spiro(bicyclo[2.2.1]-heptan)-2,3-pyrrolidin-5-carbonsäure; Spiro(bicyclo-[2.2.2]octan)-2,3-pyrrolidin-5-carbonsäure; 2-Azatricyclo[4.3.0.$1^{6,9}$]decan-3-carbonsäure; Decahydrocyclohepta-[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxypyrrolidin-2-carbonsäure; die alle gegebenenfalls substituiert sein können (siehe folgende Formeln):

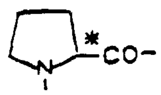 ; 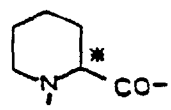 ;

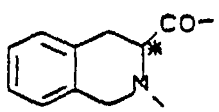 ; 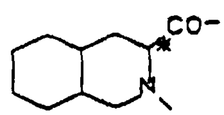 ; 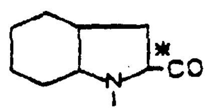 ; 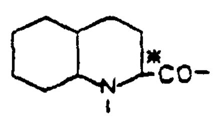 ;

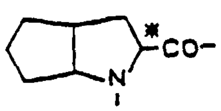 ; 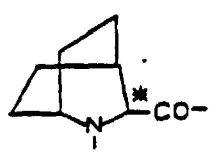 ; 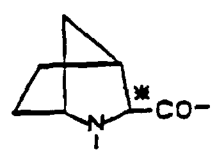 ; 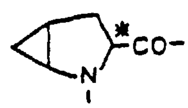 ;

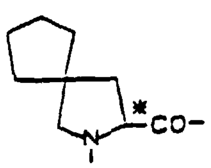 ; 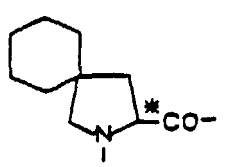 ; 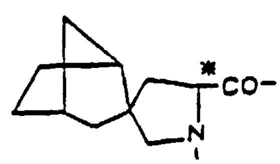 ;

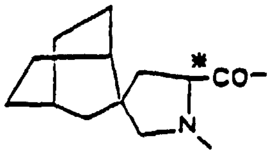 ; 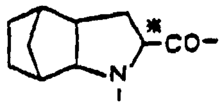 ; 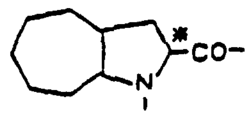 ;

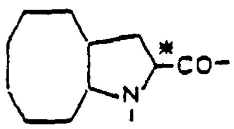 ; 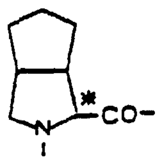 ; 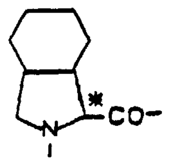 ;

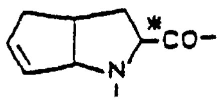 ; 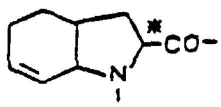 ; 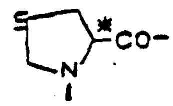 ;

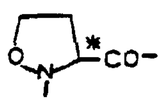 ; 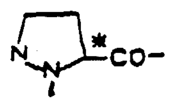 ; 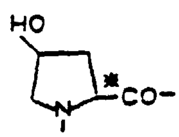 ;

Die oben genannten Resten zugrundeliegenden Heterocyclen sind beispielsweise bekannt aus

US-A 4,344,949; US-A 4,374,847; US-A 4,350,704;
EP-A 29,488; EP-A 31,741; EP-A 46,953; EP-A 49,605;
EP-A 49,658; EP-A 50,800; EP-A 51,020; EP-A 52,870;
EP-A 79,022; EP-A 84,164; EP-A 89,637; EP-A 90,341;
EP-A 90,362; EP-A 105,102; EP-A 109,020; EP-A 111,873;
EP-A 271,865 und EP-A 344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch als Ester bzw. Amide vorliegen, wie z. B. Methylester, Ethylester, Isopropylester, Isobutylester, tert.-Butylester, Benzylester, Ethylamid, Semicarbazid oder ω-Amino-($C_4$-$C_8$)-alkylamid.

Funktionelle Gruppen der Aminosäuren, Iminosäuren und Dipeptide können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z($NO_2$), Z($Hal_n$), Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin, Ethanolamin oder Tris-(2-hydroxy-ethyl)-amin.

Verbindungen der allgemeinen Formel I, welche basische Gruppen, z. B. eine Aminogruppe, eine Amidinogruppe oder eine Guanidinogruppe enthalten, bilden mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure, Methansulfonsäure oder p-Toluolsulfonsäure Salze.

Die erfindungsgemäßen Verbindungen der allgemeinen Formel I können optisch aktive Kohlenstoffatome enthalten und somit in Form reiner Enantiomerer oder in Form von Enantiomerengemischen vorliegen. Sowohl reine

Enantiomere und Enantiomerengemische als auch Diastereomere und Diastereomerengemische sind Gegenstand der vorliegenden Erfindung.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Bevorzugt sind Verbindungen der Formel I, worin **Y** $-(CH_2)_mCO-$, wobei m für 1 oder 2 steht, oder $-CHR^sCO-$, wobei $R^s$ für die Seitenkette der Aminosäuren Alanin, Valin, Phenylalanin, Tyrosin, Leucin, Isoleucin, Tryptophan, Lysin, Histidin, Asparagin, Glutamin oder Phenylglycin steht, oder

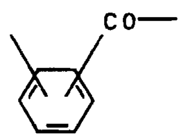

bedeutet;

**r** für 1 steht;
**W** $-COW^1$ bedeutet;
$\mathbf{z^2}$

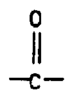

bedeutet;
$\mathbf{W^1}$ Hydroxy, $(C_1-C_4)$-Alkoxy, insbesondere Methoxy, Ethoxy, 2-Propyloxy, Isobutyloxy oder tert.-Butyloxy, oder Benzyloxy bedeutet;
**R** Wasserstoff bedeutet;
$\mathbf{R^1}$ $-(CH_2)_n-NH-X$, wobei n für eine ganze Zahl von 1 bis 5 steht, $-(CH_2)_p-C_6H_4-(CH_2)_q-NH-X$ oder $-(CH_2)_p-C_6H_4-(CH_2)_q--C(=NX)-NH_2$, wobei p und q unabhängig voneinander für 0 oder 1 stehen, bedeutet;
**X** Wasserstoff, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl oder einen Rest der Formel

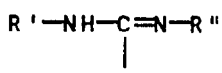

bedeutet, worin R' und R" unabhängig voneinander für Wasserstoff, Trifluorethyl, $(C_1-C_6)$-Alkylcarbonyl, $(C_1-C_6)$-Alkoxycarbonyl, $(C_1-C_{18})$-Alkylcarbonyloxy-$(C_1-C_6)$-alkoxycarbonyl oder $(C_6-C_{14})$-Aryl-$(C_1-C_6)$-alkoxycarbonyl stehen;
$\mathbf{R^2}$ Wasserstoff bedeutet;
$\mathbf{R^3}$ $-CO-NH-R^4$ bedeutet, wobei $-NH-R^4$ für den Rest einer α-Aminosäure, deren ω-Amino-$(C_2-C_8)$-alkylamid oder deren $(C_1-C_8)$-Alkyl- oder Benzylester steht, oder wobei
$R^4$ Methyl bedeutet, das durch eine Aminosäureseitenkette und durch einen Rest aus der Reihe $-SO_2-OH$, $-SO_2-NHR^9$, Tetrazolyl substituiert ist.

Für $-NH-R^4$ stehende Reste von α-Aminosäuren sind dabei besonders bevorzugt der Valin-, Lysin-, Phenylalanin-, Phenylglycin- oder 4-Chlorphenylglycin-Rest. Steht $-NH-R^4$ dabei für einen Ester einer dieser α-Aminosäuren, so ist der Methyl-, Ethyl-, Isopropyl-, Isobutyl-, tert.-Butyl-ester oder Benzylester bevorzugt.

Verbindungen der Formel I können hergestellt werden durch Fragmentkondensation einer Verbindung der allgemeinen Formel III

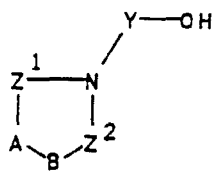

(III)

mit einer Verbindung der allgemeinen Formel IV

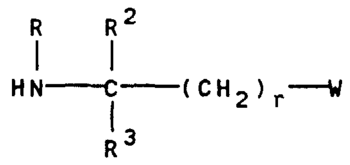

(IV)

wobei die Reste $Z^1$, $Z^2$, A, B, R, $R^2$, $R^3$, Y und W sowie r wie oben angegeben definiert sind.

Die Ausgangsverbindungen der allgemeinen Formel IV werde in der Regel vom C-terminalen Ende her stufenweise aufge baut. Zur Kondensation der Verbindungen der allgemeinen Formel III mit denen der allgemeinen Formel IV verwendet man vorteilhafterweise die an sich bekannten Kupplungsmethoden der Peptidchemie (siehe z. B. Houben-Weyl, Meth oden der organischen Chemie, Band 15/1 und 15/2, Stuttgart, 1974). Dazu ist es in der Regel nötig, daß in $R^1$ und $R^3$ und W enthaltene Aminogruppen durch reversible Schutzgruppen während der Kondensation geschützt werden. Gleiches gilt für die Carboxylgruppen der Verbindungen der Formel IV, die bevorzugt als $(C_1$-$C_6)$-Alkyl, Benzyloder tert.-Butylester vorliegen. Ein Aminogruppen-Schutz erübrigt sich, wenn die zu generierenden Aminogruppen noch als Nitro- oder Cyanogruppen vorliegen und erst nac der Kupplung durch Hydrierung gebildet werden. Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Die Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können, wenn B für $-CH_2-$ steht und $Z^1$ und $Z^2$ für -CO- stehen, wie folgt erhalten werden:

Durch Reaktion von Bernsteinsäurederivaten der allgemeinen Formel V oder deren Estern

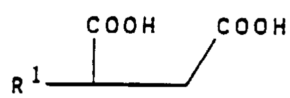

(V)

oder von Ethantricarbonsäurederivaten der allgemeinen Formel Va oder deren Estern, wobei $R^1$ wie oben angegeben definiert ist,

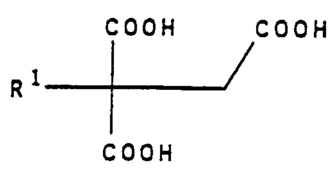

(Va)

mit Aminosäuren der allgemeinen Formel VI oder bevorzugt deren Methyl-, Ethyl-, Benzyl- oder tert.-Butylestern,

$$H_2N\text{-}Y\text{-}OH \qquad \text{(VI)}$$

wobei Y wie oben angegeben definiert ist (siehe z. B. Houben-Weyl, Methoden der organischen Chemie, Band 11/2

und E5).

Während der Cyclisierung können Guanidinogruppen durch Schutzgruppen, wie $NO_2$ oder Mtr, blockiert werden. Ebenso müssen Aminogruppen oder Amidinogruppen in der Seitenkette in geschützter Form (beispielsweise als Boc-oder Z-Derivat) oder noch als $NO_2$- oder Cyanofunktion vorliegen, die später zur Aminogruppe reduziert oder im Falle der Cyanogruppe auch in die Amidinogruppe umgewandelt werden kann.

Die Ausgangsverbindungen der allgemeinen Formel III können, wenn B für Sauerstoff, $Z^1$ und $Z^2$ für -CO- stehen, erhalten werden durch Umsetzung von Verbindungen der allgemeinen Formel X,

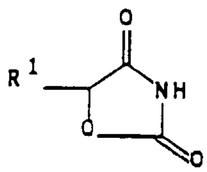

(X)

die nach literaturbekannten Verfahren hergestellt werden können (s. z.B. J. W. Clarke-Lewis, Chem. Rev. 58 (1958), 63; R. L. Dow et al, J. Med. Chem. 34 (1991), 1538 und dort zitierte Literatur; EP-A 428 312), mit Verbindungen der allgemeinen Formel XI

$$R^{10}\text{-Y-OCH}_3 \qquad \text{(XI)}$$

wobei $R^1$ und Y wie oben angegeben definiert sind und $R^{10}$ eine geeignete Abgangsgruppe darstellt, und anschließende Verseifung der Esterfunktionen.

Die Ausgangsverbindungen der allgemeinen Formel III können, wenn B für $-CH_2-$ steht und $Z^1$ für -CO- und $Z^2$ für $-CH_2-$ steht, wie folgt erhalten werden:

Durch N-Alkylierung von Verbindungen der allgemeinen Formel XV

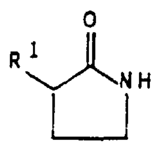

(XV)

mit Verbindungen der allgemeinen Formel XI, wobei die Verbindungen der allgemeinen Formel XV durch C-Alkylierung von am Stickstoff geschütztem 2-Pyrrolidinon mit Verbindungen der allgemeinen Formel XVI,

$$R^1\text{-}R^{10} \qquad \text{(XVI)}$$

worin $R^{10}$ eine geeignete Abgangsgruppe darstellt, erhältlich sind. Entsprechende Alkylierungsreaktionen an Pyrrolidinonen sind beispielsweise beschrieben bei M. A. E. Bowman et al., Org. Prep. Proced. Int. 22 (1990) 636; C. H. Kochbar et al., J. Org. Chem. 50 (1985) 3019; J. D. Stewart et al., J. Org. Chem. 52 (1987) 2113; T. J. Hagen, Synlett (1990) 63.

Bei allen Reaktionsschritten müssen gegebenenfalls freie funktionelle Gruppen durch geeignete reversible Schutzgruppen blockiert werden, die später in geeigneter Weise wieder abgespalten werden.

Für die Guanylierung und Nitroguanylierung der Aminoverbindungen können folgende Reagentien verwendet werden:

1. O-Methylisoharnstoff (S. Weiss und H. Krommer, Chemiker Zeitung 98 (1974) 617 - 618),
2. S-Methylisothioharnstoff R.F. Borne, M.L. Forrester und I.W. Waters, J. Med. Chem. 20 (1977) 771 - 776),
3. Nitro-S-methylisothioharnstoff (L.S. Hafner und R.E. Evans, J. Org. Chem. 24 (1959) 1157),
4. Formamidinsulfonsäure (K. Kim, Y.-T. Lin und H.S. Mosher, Tetrahedron Lett. 29 (1988) 3183 - 3186),
5. 3,5-Dimethyl-l-pyrazolyl-formamidinium-nitrat (F.L. Scott, D.G. O'Donovan und J. Reilly, J. Amer. Chem. Soc. 75 (1953) 4053 - 4054),

6. N,N'-Di-tert.-butyloxycarbonyl-S-methyl-isothioharnstoff (R.J. Bergeron und J.S. McManis, J. Org. Chem. 52 (1987) 1700 - 1703),

7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers, A. Widding, P. Andrews, H.-P. Schulz und H. Thomas, Arzneim. Forsch./ Drug Res. 34 (1984) 531 - 542).

Amidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließende Aminolyse hergestellt werden (G. Wagner, P. Richter und Ch. Garbe, Pharmazie 29, (1974) 12 - 15). Eine weitere Methode, Amidine herzustellen, ist die Anlagerung von $H_2S$ an die Cyanogruppe, gefolgt von einer Methylierung des entstandenen Thioamids und anschließender Umsetzung mit Ammoniak (DDR-Patent Nr. 235 866).

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z. B. in Form von Suppositorien oder parenteral, z. B. in Form von Injektions- oder Infusionslösungen oder Mikrokapseln, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln oder Implantate eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihre pharmakologisch annehmbaren Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüber hinaus lassen sich die Verbindungen beispielsweise auch mit nootrop wirksamen Substanzen, wie z. B. Piracetam, oder ZNS-aktiven Substanzen, wie Pirlindol, Sulpirid etc. kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis kann, insbesondere bei der Applikation größerer Mengen, in mehrere, z. B. 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer pharmazeutisch akzeptablen Salze pro Dosis.

Die erfindungsgemäßen neuen Heterocyclen der Formel I haben die Fähigkeit, die Bindung von Fibrinogen, Fibronectin und des von Willebrand-Faktors an Integrinrezeptoren zu hemmen. Sie beeinflussen auf diese Weise die Zell-Zell- und Zell-Matrix-Wechselwirkung und können so die Bildung von Blutplättchenthromben verhindern. Integrine sind Zellmembran-Glykoproteine und vermitteln Zelladhäsion durch Wechselwirkung mit einer Vielzahl extrazellulärer

Proteine wie Fibronectin, Laminin, Kollagen, Vitronectin und von Willebrand-Faktor oder mit anderen Zellmembranproteinen wie z. B. ICAM-1. Ein wichtiger Rezeptor aus der Integrinfamilie ist das auf Blutplättchen lokalisierte Glykoprotein IIb/IIIa (Fibrinogen-Rezeptor) - ein Schlüsselprotein der Plättchen-Plättchen-Wechselwirkung und Thrombenbildung. Ein zentrales Fragment in der Rezeptorerkennungssequenz dieser Proteine ist das Tripeptid Arg-Gly-Asp (E. Ruoslahti und M.D. Pierschbacher, Science 238 (1987) 491 - 497; D.R. Phillips, I.F. Charo, L.V. Parise und L.A. Fitzgerald, Blood 71 (1988) 831 - 843).

Eine Anwendung finden die Heterocyclen der allgemeinen Formel I daher zur Prophylaxe und Therapie von arteriellen Gefäßerkrankungen wie akutem Myokardinfarkt in Kombination mit Lysetherapie, post-Infarktbehandlung, Sekundärprävention des Myokardinfarktes, Reocclusionsprophylaxe nach Lyse und Dilatation, instabiler Angina Pectoris, transitorischer ischämischer Attacken, Schlaganfall, koronarer Bypass-Operation und Reocclusionsprophylaxe des Bypasses, Lungenembolie, peripherer arterieller Verschlußkrankheiten, dissezierendem Aneurysma, zur Therapie venöser und mikrozirkulatorischer Gefäßerkrankungen wie tiefer Venenthrombose, disseminierter intravaskulärer Gerinnung, post-operativem und post-partum Trauma, chirurgischem oder infektiösem Schock, Septicämie, zur Therapie bei Erkrankungen mit hyperreagiblen Thrombozyten, thrombotische thrombozytopenische Purpura, Preeklampsie, prämenstruelles Syndrom, Dialyse, extrakorporale Zirkulation; eine weitere Anwendung ist bei Entzündungen und bei der Behandlung von Tumoren gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen. Verwendet werden gelfiltrierte Blutplättchen aus humanem Spenderblut, die mit ADP oder Thrombin aktiviert werden.

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben wird der Ki-Wert der Bindungshemmung von $^{125}$I-Fibrinogen nach Stimulierung mit ADP (10 µM). (Literatur: J.S. Bennett u. G. Vilaire, J. Clin. Invest. 64 (1979) 1393 - 1401; E. Kornecki et al., J. Biol. Chem. 256 (1981), 5695 - 5701; G.A. Marguerie et al., J. Biol. Chem. 254 (1979) 5357 - 5363; G.A. Marguerie et al. J. Biol. Chem. 255 (1980 154 - 161).

Als funktioneller Test wird die Hemmung der Aggregation gelfiltrierter Human-Thrombocyten nach ADP- bzw. Thrombin-Stimulierung durch die erfindungsgemäßen Verbindungen gemessen. Angegeben ist der $IC_{50}$-Wert der Hemmung (Literatur: G.A. Marguerie et al., J. Biol. Chem. 254 (1979), 5357-5363).

Bei der Prüfung der Hemmung der Fibrinogenbindung und der Hemmung der Aggregation werden für die Verbindungen der nachfolgenden Beispiele folgende Ergebnisse erhalten:

| Beispiel | Plättchenaggregationshemmung | | Hemmung der Fibrinogenbindung |
|---|---|---|---|
| | ADP (µM) | Thrombin (µM) | Ki (µM) |
| 1 | 0.2 | 0.09 | 0.043 |
| 12 | 100 | 80 | |
| 13 | 100 | 100 | |
| 17 | 100 | 100 | |
| 18 | 100 | 70 | |
| 14 | 20 | 35 | |
| 15 | 200 | 200 | |
| 16 | 20 | 9 | |
| 19 | 4 | 1 | |

**BEISPIELE**

Beispiel 1

{3-[4-(Amino-imino-methyl)-benzyl]-2,5-dioxo-pyrrolidin-1-yl}-acetyl-L-aspartyl-L-phenylglycin

a) Synthese von 4-(4-Cyanobenzyl)-3,3-bis-ethoxycarbonylbuttersäureethylester

24.6 g 1,1,2-Ethantricarbonsäuretriethylester werden in 500 ml abs. Ethanol gelöst und in Portionen mit 11.2 g Kalium-tert.-butylat versetzt. Zu dieser Mischung werden 19.6 g p-(Brommethyl)-benzonitril in Portionen zugegeben. Man erwärmt für 5 Stunden auf ca. 60°C. Dann läßt man auf Raumtemperatur abkühlen, filtriert vom Niederschlag ab und engt das Filtrat im Vakuum ein. Der verbleibende ölige Rückstand wird an Kieselgel mit Dichlormethan/Methanol/Wasser/Essigsäure (100/10/1/1) chromatographiert. Die Produktfraktionen werden vereinigt und im Vakuum zur Trockene eingeengt. Es verbleibt ein Öl, das langsam kristallisiert.
Ausbeute: 28.7 g
MS(CI): 362 [M+H]$^+$
NMR($CDCl_3$): $\delta$ = 1.25 ppm (m, 9H); 2.82 ppm (s, 2H); 3.45 ppm (s, 2H); 4.20 ppm (m, 6H); 7.25 ppm (d, 2H); 7.55 ppm (d, 2H).

b) Synthese von 4-[4-(Amino-imino-methyl)-benzyl]-3,3-bis-ethoxycarbonyl-buttersäureethylester-hydrochlorid

28.7 g 4-(4-Cyanobenzyl)-3,3-bis-ethoxycarbonyl-buttersäureethylester werden in 900 ml abs. Ethanol gelöst und in einem Trockeneisbad auf -5°C abgekühlt. In diese Lösung wird trockenes HCl-Gas eingeleitet, wobei man die Temperatur immer unter 0°C hält. Nach ca. 4 h läßt die stark exotherme Reaktion nach und man läßt die Reaktionsmischung bei 0°C über Nacht stehen. Dann wird das Lösungsmittel am Rotationsverdampfer im Vakuum abgezogen und der ölige Rückstand in 200 ml trockenem Isopropanol gelöst und vorsichtig mit $NH_3$/Isopropanol versetzt bis die Mischung pH 8 erreicht hat. Die Mischung wird 3 h bei 60 - 70°C gerührt und über Nacht bei Raumtemperatur stehengelassen. Die erkaltete Lösung wird filtriert und das Filtrat im Vakuum eingeengt und der ölige Rückstand mit Diethylether verrieben. Das feste Präzipitat wird abgesaugt und mit Diethylether gewaschen. Nach Trocknung im Exsiccator verbleiben 25.47 g.
MS(FAB): 379.2 [M+H]$^+$
NMR($CDCl_3$): $\delta$ = 1.25 ppm (m, 9H); 2.75 ppm (s, 2H); 3.40 ppm (s, 2H); 4.18 ppm (m, 6H); 7,28 ppm (d, 2H); 7.88 ppm (d, 2H); 8.43 ppm (br s, 2H); 9.33 ppm (br s, 2 H).

c) Synthese von 4-[4-(Amino-imino-methyl)-benzyl]-3,3-bis-ethoxycarbonyl-buttersäure-hydrochlorid

11.34 g 4-[4-(Amino-imino-methyl)-benzyl]-3,3-bis-ethoxycarbonyl-buttersäureethylester-hydrochlorid werden in 250 ml 6N HCl 1 h bei 40°C und 2 h bei 80°C gerührt. Dann läßt man über Nacht bei Raumtemperatur stehen und engt im Vakuum bis zur Trockene ein. Der Rückstand wird in wenig Wasser aufgenommen und gefriergetrocknet.
Ausbeute: 10.5 g
MS(FAB): 351.2 [M+H]$^+$

d) Synthese von {4-[4-(Amino-imino-methyl)-benzyl]-3,3-bis-ethoxycarbonyl-butyryl}-glycin-methylester-hydrochlorid

10.5 g 4-[4-(Amino-imino-methyl)-benzyl]-3,3-bis-ethoxycarbonyl-buttersäure-hydrochlorid werden in 500 ml Dimethylformamid gelöst. Dann werden nacheinander 3.75 g Glycinmethylester-hydrochlorid, 9.83 g O-[(Cyanethoxycarbonylmethylen)-amino]-N,N,N',N'-tetramethyluroniumtetrafluoroborat (TOTU) und 11,5 ml N-Ethylmorpholin zugegeben. Man rührt die Mischung 24 h und zieht dann das Lösungsmittel im Vakuum ab. Der ölige Rückstand wird an Kieselgel mit Dichlormethan/Methanol/Wasser/Essigsäure (100/10/1/1) chromatographiert. Die das Produkt enthaltenden Fraktionen werden vereinigt und eingeengt.
Ausbeute: 7.8 g
MS(FAB): 422.2

e) Synthese von {3-[4-(Amino-imino-methyl)-benzyl]-2,5-dioxo-pyrrolidin-1-yl}-essigsäure-hydrochlorid

4 g {4-[4-(Amino-imino-methyl)-benzyl]-3,3-bis-ethoxycarbonyl-butyryl}-glycin-methylester-hydrochlorid werden in 100 ml konz. HCl 30 min unter Rückfluß erhitzt, dann im Vakuum zur Trockene eingeengt, zweimal mit 10 ml Wasser nachdestilliert und im Hochvakuum getrocknet.
Ausbeute: 3 g
MS(FAB): 290.1 [M+H]$^+$

f) Synthese von {3-[4-(Amino-imino-methyl)-benzyl]-2,5-dioxo-pyrrolidin-1-yl}-acetyl-L-($\beta$-tert.-butyl)aspartyl-L-phenylglycin-tert.-butylester

1,74 g {3-[4-(Amino-imino-methyl)-benzyl]-2,5-dioxo-pyrrolidin-1-yl}-essigsäure-hydrochlorid werden in 120 ml DMF gelöst und nacheinander mit 2.49 g H-Asp(OtBu)-Phg-OtBu, 2.95 g TOTU und 3.45 ml N-Ethylmorpholin versetzt. Man rührt 24 h und engt dann die Mischung zur Trockene ein. Der verbleibende ölige Rückstand wird an Kieselgel mit Dichlormethan/Methanol/Wasser/Essigsäure (100/10/1/1) chromatographiert. Die vereinigten substanzhaltigen Fraktionen werden eingeengt. Es verbleiben 0.98 g.
MS(FAB): 650.3 [M+H]$^+$

g) Synthese von {3-[4-(Amino-imino-methyl)-benzyl]-2,5-dioxo-pyrrolidin-1-yl}-acetyl-L-aspartyl-L-phenylglycin

980 mg {3-[4-(Amino-imino-methyl)-benzyl]-2,5-dioxo-pyrrolidin-1-yl}-acetyl-L-($\beta$-tert.-butyl)aspartyl-L-phenylglycin-tert.-butylester werden mit 20 ml Trifluoressigsäure versetzt und nach 20 min. Stehenlassen zur Trockene eingeengt. Der Rückstand wird dreimal mit etwas Diethylether nachdestilliert, dann in wenig Wasser aufgenommen und gefriergetrocknet.
Ausbeute: 398 mg
MS(FAB): 538.2 $[M+H]^+$
$^1$H-NMR ($D_2O$): $\delta$ = 2.51 - 3.51 ppm (m, 8H), 4.10 - 4.35 ppm (m, 2H), 5.45 (d, 1H), 7.30 - 7.80 ppm (m, 9H)
Aminosäureanalyse: Asp: 0.99 (1); Gly: 1.00 (1); Phg: 1.01 (1).

Analog werden erhalten:

Beispiel 2

{3-[4-(Amino-imino-methyl)-benzyl]-2,5-dioxo-pyrrolidin-1-yl}-acetyl-L-aspartyl-L-phenylalanin

Beispiel 3

{3-[4-(Amino-imino-methyl)-benzyl]-2,5-dioxo-pyrrolidin-1-yl}-acetyl-L-aspartyl-L-valin

Beispiel 4

{3-[4-(Amino-imino-methyl)-phenyl]-2,5-dioxo-pyrrolidin-1-yl}-acetyl-L-aspartyl-L-phenylglycin

Beispiel 5

2-{3-[4-(Amino-imino-methyl)-benzyl]-2,5-dioxo-pyrrolidin-1-yl}-propionyl-L-aspartyl-L-phenylglycin

Beispiel 7

{5-[4-(Amino-imino-methyl)-benzyl]-2,4-dioxo-[1,3]oxazolidin-3-yl}-acetyl-L-aspartyl-L-phenylglycin

Beispiel 9

[3-(3-Guanidino-propyl)-2,5-dioxo-pyrrolidin-1-yl]-acetyl-L-aspartyl-L-phenylglycin

Beispiel 10

3-[3-(3-Amino-propyl)-2,5-dioxo-pyrrolidin-1-yl]-benzoyl-L-aspartyl-L-phenylglycin

Beispiel 11

<{{3-[4-(Amino-imino-methyl)-benzyl]-2,5-dioxopyrrolidin-1-yl}-acetyl-L-aspartylamido}-phenyl-methylsulfonyl>-harnstoff

Beispiel 12

{3-[4-(Amino-imino-methyl)-benzyl]-2,5-dioxo-pyrrolidin-1-yl}-acetyl-L-tryptophan-methylester

403 mg {3-[4-(Amino-imino-methyl)-benzyl]-2,5-dioxopyrrolidin-1-yl}-essigsäure-hydrochlorid werden nach dem oben beschriebenen Verfahren mit 356.6 mg L-Tryptophanmethylester-hydrochlorid gekuppelt. Nach Aufarbeitung und chromatographischer Reinigung erhält man 270 mg {3-[4-(Amino-imino-methyl)-benzyl]-2,5-dioxo-pyrrolidin-1-yl}-acetyl-L-tryptophan-methylester.
MS(FAB): 490.2 $[M+H]^+$

Beispiel 13

{3-[4-(Amino-imino-methyl)-benzyl]-2,5-dioxo-pyrrolidin-1-yl}-acetyl-D,L-homophenylalanin-methylester

403 mg {3-[4-(Amino-imino-methyl)-benzyl]-2,5-dioxopyrrolidin-1-yl}-essigsäure-hydrochlorid werden nach

dem oben beschriebenen Verfahren mit 270 mg D,L-Homophenylalanin-methylester gekuppelt. Nach Aufarbeitung und chromatographischer Reinigung erhält man 343 mg {3-[4-(Amino-imino-methyl)-benzyl]-2,5-dioxo-pyrrolidin-1-yl}-acetyl-D,L-homophenylalanin-methylester.
MS(FAB): 465.2 $[M+H]^+$

Beispiel 14

{3-[4-(Amino-imino-methyl)-benzyl]-2,5-dioxo-pyrrolidin-1-yl}-acetyl-L-(β-isopropyl)aspartyl-L-phenylglycinisopropylester

412 mg {3-[4-(Amino-imino-methyl)-benzyl]-2,5-dioxopyrrolidin-1-yl}-essigsäure-hydrochlorid werden nach dem oben beschriebenen Verfahren mit 500 mg L-(ß-Isopropyl)aspartyl-L-phenylglycin-isopropylester gekuppelt. Nach Aufarbeitung und chromatographischer Reinigung erhält man 330 mg {3-[4-(Amino-imino-methyl)-benzyl]-2,5-dioxo-pyrrolidin-1-yl}-acetyl-L-(β-isopropyl)aspartyl -L-phenylglycin-isopropylester.
MS(FAB): 622.4 $[M+H]^+$

Beispiel 15

3-(R,S)-3-<{3-[4-(Amino-imino-methyl)-benzyl]-2,5-dioxopyrrolidin-1-yl}-acetamido>-3-(3-hydroxyphenyl)-propionsäuremethylester

500 mg {3-[4-(Amino-imino-methyl)-benzyl]-2,5-dioxopyrrolidin-1-yl}-essigsäure-hydrochlorid werden nach dem oben beschriebenen Verfahren mit 400 mg 3-(R,S)-3-Amino-3-(3-hydroxyphenyl)-propionsäuremethylester-hydrochlorid gekuppelt. Nach Aufarbeitung und chromatographischer Reinigung erhält man 78 mg 3-(R,S)-3-<{3-[4-(Aminoimino-methyl)-benzyl]-2,5-dioxo-pyrrolidin-1-yl}-acetamido>-3-(3-hydroxyphenyl)-propionsäuremethylester.
MS(ES): 467.2 $[M+H]^+$

Beispiel 16

3-(R,S)-3-<{3-[4-(Amino-imino-methyl)-benzyl]-2,5-dioxopyrrolidin-1-yl}-acetamido>-3-(3-hydroxyphenyl)-propionsäure

50 mg 3-(R,S)-3-<{3-[4-(Amino-imino-methyl)-benzyl]-2,5-dioxo-pyrrolidin-1-yl}-acetamido>-3-(3-hydroxyphenyl)-propionsäuremethylester werden 1 h mit 5 ml konzentrierter Salzsäure bei Raumtemperatur stehengelassen. Dann wird im Vakuum zur Trockene eingeengt, der Rückstand in wenig Wasser aufgenommen und gefriergetrocknet. Man erhält 22 mg 3-(R,S)-3-<{3-[4-(Amino-imino-methyl)-benzyl]-2,5-dioxo-pyrrolidin-1-yl}-acetamido>-3-(3-hydroxyphenyl)-propionsäure.
MS(ES): 453.1 $[M+H]^+$

Beispiel 17

{3-[4-(Amino-imino-methyl)-benzyl]-2,5-dioxo-pyrrolidin-1-yl}-acetyl-D,L-homophenylalanin

45 mg {3-[4-(Amino-imino-methyl)-benzyl]-2,5-dioxopyrrolidin-1-yl}-acetyl-D,L-homophenylalanin-methylester werden 1 h mit 5 ml konzentrierter Salzsäure bei Raumtemperatur stehengelassen. Dann wird im Vakuum zur Trockene eingeengt, der Rückstand in wenig Wasser aufgenommen und gefriergetrocknet.
Man erhält 24 mg {3-[4-(Amino-imino-methyl)-benzyl]-2,5-dioxo-pyrrolidin-1-yl}-acetyl-D,L-homophenylalanin. MS (FAB): 451.2 $[M+H]^+$

Beispiel 18

{3-[4-(Amino-imino-methyl)-benzyl]-2,5-dioxo-pyrrolidin-1-yl}-acetyl-L-tryptophan

204 mg {3-[4-(Amino-imino-methyl)-benzyl]-2,5-dioxopyrrolidin-1-yl}-acetyl-L-tryptophan-methylester werden 3 min mit 10 ml 6N HCl erwärmt, dann zur Trockene eingedampft und zweimal mit Wasser nachdestilliert. Der Rückstand wird in wenig Wasser aufgenommen und gefriergetrocknet. Man erhält 68 mg {3-[4-(Amino-imino-methyl)-benzyl]-2,5-dioxo-pyrrolidin-1-yl}-acetyl-L-tryptophan. MS(FAB): 476.2 $[M+H]^+$

Beispiel 19

{3-[Piperidin-4-yl]-2,5-dioxo-pyrrolidin-1-yl}-acetyl-L-aspartyl-L-phenylglycin

a) Synthese von 4-(4-Pyridyl)-3,3-bis-ethoxycarbonylbuttersäureethylester

24.6 g 1,1,2-Ethantricarbonsäuretriethylester werden in 900 ml abs. Ethanol gelöst und in Portionen mit 38.6 g Kalium-tert.-Butylat versetzt. Zu dieser Mischung werden 29.8 g 4-(Chlormethyl)pyridin-hydrochlorid suspendiert in 400 ml abs. Ethanol in Portionen zugegeben. Man erwärmt für 5 h auf ca. 50°C und läßt über Nacht bei Raumtemperatur stehen. Dann filtriert man vom Niederschlag ab und engt das Filtrat im Vakuum ein. Der verbleibende ölige Rückstand wird an Kieselgel mit Ethylacetat chromatographiert. Die sauberen Fraktionen werden vereinigt und im Vakuum zur Trockene eingeengt. Es verbleibt ein Öl, das langsam kristallisiert.
Ausbeute: 19.7 g
MS(CI): 338 $[M+H]^+$
NMR($CDCl_3$): 1.25 ppm (m, 9H); 2.84 ppm (s, 2H); 3.40 ppm (s, 2H): 4.20 ppm (m, 6H); 7.08 ppm (dd, 2H); 8.50 ppm (dd, 2H).

b) Synthese von 4-(4-Pyridyl)-3,3-bis-ethoxycarbonylbuttersäure

8.75 g 4-(4-Pyridyl)-3,3-bis-ethoxycarbonyl-buttersäureethylester werden in 500 ml 6N HCl 2 h bei 50-60°C gerührt. Dann engt man im Vakuum ein und läßt nochmals mit 500 ml 6N HCl 2h bei 50-60°C reagieren. Dann engt man im Vakuum bis zur Trockne ein und destilliert zweimal mit wenig Toluol nach. Der Rückstand wird in wenig Wasser aufgenommen und gefriergetrocknet.
Ausbeute: 9.31 g (enthält noch etwas Triester)
MS(FAB): 310.1 $[M+H]^+$

c) Synthese von [4-(4-Pyridyl)-3,3-bis-ethoxycarbonylbutyryl]-glycin-methylester

9.27 g 4-(4-Pyridyl)-3,3-bis-ethoxycarbonyl-buttersäure werden in 600 ml Dimethylformamid gelöst. Dann werden nacheinander 3.69 g Glycinmethylesterhydrochlorid, 8.78 g TOTU und 11.5 ml N-Ethylmorpholin zugegeben. Man rührt die Mischung 24 h und zieht dann das Lösungsmittel im Vakuum ab. Der ölige Rückstand wird an Kieselgel mit Ethylacetat chromatographiert. Die das Produkt enthaltenen Fraktionen werden vereinigt und eingeengt.
Ausbeute: 6.6 g
MS(FAB): 381.1 $[M+H]^+$

d) Synthese von [3-(4-Pyridyl)-2,5-dioxo-pyrrolidin-1-yl]-essigsäure

3.58 g [4-(4-Pyridylyl)-3,3-bis-ethoxycarbonyl-butyryl]-glycin-methylester werden in 1000 ml. konz. HCl 30 min refluxiert, dann im Vakuum zur Trockne eingeengt, fünfmal mit Toluol nachdestilliert und im Hochvakuum getrocknet. Ausbeute: 2,75 g
MS(DCI): 249 $[M+H]^+$

e) Synthese von [3-(Piperidin-4-yl)-2,5-dioxo-pyrrolidin-1-yl]-essigsäure

2.8 g [3-(4-Pyridyl)-2,5-dioxo-pyrrolidin-1-yl]-essigsäure werden in 160 ml Essigsäure suspendiert und mit 100 mg 5% Rhodium auf Kohle als Katalysator 24 h bei 100°C und 150 bar Wasserstoffdruck im Autoklaven hydriert. Dann wird der Katalysator abfiltriert und das Filtrat im Vakuum zur Trockne eingeengt. Das so erhaltene Produkt wird in wenig Wasser aufgenommen und gefriergetrocknet.
Ausbeute: 2.3 g
MS(ES): 255 $[M+H]^+$

f) Synthese von {3-[(1-tert.-Butyloxycarbonyl)-piperidin-4-yl]-2,5-dioxo-pyrrolidin-1-yl}-essigsäure

100 mg [3-(Piperidin-4-yl)-2,5-dioxo-pyrrolidin-1-yl]-essigsäure werden in 15 ml DMF gelöst und mit 87.3 mg Di-tert.-butylcarbonat und 51 µl Triethylamin versetzt. Nach 1 h wird das Lösungsmittel im Vakuum abgezogen und der Rückstand zwischen Ether und Wasser verteilt. Die Etherphase wird über Natriumsulfat getrocknet und dann im Vakuum eingeengt. Es verbleiben 120 mg eines Öls, das sofort weiter umgesetzt wird.
MS(FAB): 377.2 $[M+Na]^+$, 355.3 $[M+H]^+$

g) Synthese von {3-[(1-tert.-Butyloxycarbonyl)-piperidin-4-yl]-2,5-dioxo-pyrrolidin-1-yl}-acetyl-L-(β-tert.-butyl)aspartyl-L-phenylglycin-tert.-butylester

110 mg {3-[(1-tert.-Butyloxycarbonyl)-piperidin-4-yl]-2,5-dioxo-pyrrolidin-1-yl}-essigsäure werden in 15 ml

DMF gelöst und nacheinander mit 128.5 mg H-Asp(OtBu)-Phg-OtBu, 101.7 mg TOTU und 120 µl N-Ethylmorpholin versetzt. Man rührt 24 h und engt dann die Mischung zur Trockne ein. Der verbleibende ölige Rückstand wird an Kieselgel mit Dichlormethan/Methanol/Wasser/Essigsäure (100/10/1/1) chromatographiert. Die vereinigten substanzhaltigen Fraktionen werden eingeengt. Es verbleiben 90 mg, die sofort weiter umgesetzt werden.
MS(ES): 715 $[M+H]^+$

h) Synthese von {3-[Piperidin-4-yl]-2,5-dioxo-pyrrolidin-1-yl}-acetyl-L-aspartyl-L-phenylglycin

90 mg {3-[(1-tert.-Butyloxycarbonyl)-piperidin-4-yl]-2,5-dioxo-pyrrolidin-1-yl}-acetyl-L-(β-tert.-butyl)aspartyl-L-phenylglycin-tert.-butylester werden in 10 ml Trifluoressigsäure gelöst, 20 min bei Raumtemperatur stehen gelassen und dann zur Trockene eingeengt. Der Rückstand wird mit etwas Diethylether zweimal nachdestilliert, in wenig Wasser gelöst und gefriergetrocknet.
Ausbeute: 60 mg
MS(FAB): 503.4 $[M+H]^+$

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe Wasser | q. s. |
| (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |

(fortgesetzt)

| | |
|---|---|
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

## Patentansprüche

1. Heterocyclen der allgemeinen Formel I,

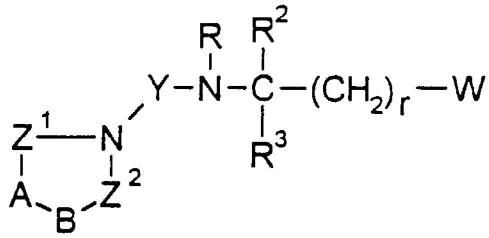

(I)

worin

$Z^1$

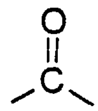 oder 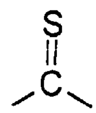

bedeutet;

$Z^2$

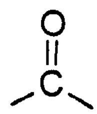 oder 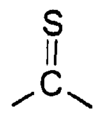

bedeutet;

Y $-(CH_2)_m$-CO-, wobei m für eine ganze Zahl von 1 bis 4 steht, oder $-CHR^s$-CO-oder

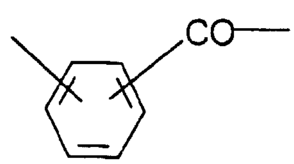

bedeutet;

r eine ganze Zahl von 0 bis 3 bedeutet;

A $-CHR^1$- bedeutet;

B $-CH_2$- oder -O- bedeutet;

W $-COW^1$, Tetrazolyl, $-SO_2$-OH oder $-SO_2-NHR^9$ bedeutet;

$W^1$ Hydroxy, $(C_1-C_{28})$-Alkoxy, $(C_6-C_{14})$-Aryl-$(C_1-C_8)$-alkoxy, $(C_6-C_{14})$-Aryloxy, Amino oder Mono- oder Di-($(C_1-C_{18})$-Alkyl)-amino bedeutet;

R Wasserstoff oder $(C_1-C_6)$-Alkyl bedeutet;

$R^1$

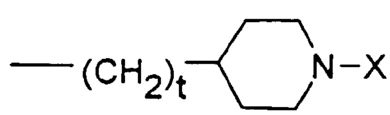

oder $-(CH_2)_n$-NH-X oder $-(CH_2)_p-C_6H_4-(CH_2)_q$-NH-X oder $-(CH_2)_p$-C(=NX)-$NH_2$ oder $-(CH_2)_p-C_6H_4-(CH_2)_q$-C

(=NX)-$NH_2$ bedeutet, worin n für eine Zahl von 1 bis 6 steht, p und q unabhängig voneinander für eine Zahl von 0 bis 3 stehen und t für eine Zahl von 0 bis 2 steht;

X Wasserstoff, ($C_1$-$C_6$)-Alkyl,($C_1$-$C_6$)-Alkylcarbonyl, ($C_1$-$C_6$)-Alkoxycarbonyl, ($C_1$-$C_{18}$)-Alkylcarbonyloxy-($C_1$-$C_6$)-alkoxycarbonyl, ($C_6$-$C_{14}$)-Arylcarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino oder einen Rest der Formel II

$$R'\text{-NH-C(=N-R'')-} \qquad (II)$$

bedeutet, wobei R' und R" unabhängig voneinander für Wasserstoff, ($C_1$-$C_6$)-Alkyl, Trifluor-($C_1$-$C_6$)-Alkyl, ($C_1$-$C_6$)-Alkoxycarbonyl, ($C_1$-$C_6$)-Alkylcarbonyl, ($C_6$-$C_{14}$)-Arylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino stehen;

$R^2$ Wasserstoff, ($C_1$-$C_4$)-Alkyl oder Phenyl bedeutet, wobei das ($C_1$-$C_4$)-Alkyl und das Phenyl unsubstituiert oder ein- oder mehrfach mit gleichen oder verschiedenen Resten aus der Reihe Hydroxy, Amino, ($C_1$-$C_4$)-Alkoxy, Imidazolyl, Indolyl, Pyrrolidinyl, Hydroxypyrrolidinyl, Phenyl oder Halogen substituiert sein kann;

$R^3$ Wasserstoff, -$COOR^4$, -CO-N($CH_3$)-$R^4$ oder -CO-NH-$R^4$ bedeutet;

$R^4$ Wasserstoff oder ($C_1$-$C_{28}$)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-(($C_1$-$C_{18}$)-alkyl)-aminocarbonyl, Amino-($C_2$-$C_{18}$)-alkylaminocarbonyl, Amino-($C_1$-$C_3$)-alkyl-phenyl-($C_1$-$C_3$)-alkylaminocarbonyl, ($C_1$-$C_{18}$)-Alkylcarbonylamino-($C_1$-$C_3$)-alkylphenyl-($C_1$-$C_3$)-alkylaminocarbonyl, ($C_1$-$C_{18}$)-Alkylcarbonylamino-($C_2$-$C_{18}$)-alkylaminocarbonyl, ($C_6$-$C_{14}$)-Aryl-($C_1$-$C_8$)-alkoxycarbonyl, Amino, Mercapto, ($C_1$-$C_{18}$)-Alkoxy, ($C_1$-$C_{18}$)-Alkoxycarbonyl, ($C_3$-$C_8$)-Cycloalkyl, Halogen, Nitro, Trifluormethyl oder durch den Rest $R^5$ substituiert sein kann, wobei

$R^5$ ($C_6$-$C_{14}$)-Aryl, ($C_6$-$C_{14}$)-Aryl-($C_1$-$C_8$)-alkyl, einen mono- oder bizyklischen 5-bis 12-gliedrigen heterocyclischen Ring, der aromatisch, teilhydriert oder vollständig hydriert sein kann und der ein, zwei oder drei gleiche oder verschiedene Heteratome aus der Reihe Stickstoff, Sauerstoff und Schwefel enthalten kann, einen Rest $R^6$ oder einen Rest $R^6$CO- bedeutet, wobei der Aryl- und unabhängig davon der Heterozyklus-Rest ein- oder mehrfach durch gleiche oder verschiedene Reste aus der Reihe ($C_1$-$C_{18}$)-Alkyl, ($C_1$-$C_{18}$)-Alkoxy, Halogen, Nitro, Amino oder Trifluormethvl substituiert sein können;

$R^6$ -$NR^7R^8$, -$OR^7$, -$SR^7$, -$SO_2$-OH, -$SO_2NHR^9$, Tetrazolyl, eine Aminosäureseitenkette, einen natürlichen oder unnatürlichen Aminosäure-, Iminosäure-, Azaaminosäure- oder einen Dipeptid-Rest sowie deren Ester und Amide bedeutet, wobei freie funktionelle Gruppen durch in der Peptidchemie übliche Schutzgruppen geschützt sein können;

$R^7$ Wasserstoff, ($C_1$-$C_{18}$)-Alkyl, ($C_6$-$C_{14}$)-Aryl-($C_1$-$C_8$)-alkyl, ($C_1$-$C_{18}$)-Alkylcarbonyl, ($C_1$-$C_8$)-Alkoxycarbonyl, ($C_6$-$C_{14}$)-Arylcarbonyl, ($C_6$-$C_{14}$)-Aryl-($C_1$-$C_8$)-alkylcarbonyl oder ($C_6$-$C_{14}$)-Aryl-($C_1$-$C_{18}$)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe ($C_1$-$C_8$)-Alkyl, ($C_1$-$C_8$)-Alkoxy, Halogen, Nitro, Amino und Trifluormethyl substituiert sein können, einen natürlichen oder unnatürlichen Aminosäure-, Iminosäure-, Azaaminosäure- oder einen Dipeptid-Rest bedeutet;

$R^8$ Wasserstoff, ($C_1$-$C_{18}$)-Alkyl, ($C_6$-$C_{14}$)-Aryl oder ($C_6$-$C_{14}$)-Aryl-($C_1$-$C_8$)-alkyl, bedeutet;

$R^9$ Wasserstoff, Aminocarbonyl, ($C_1$-$C_{18}$)-Alkylaminocarbonyl, ($C_3$-$C_8$)-Cycloalkylaminocarbonyl, ($C_1$-$C_{18}$)-Alkyl oder ($C_3$-$C_8$)-Cycloalkyl bedeutet;

$R^s$ eine Aminosäureseitenkette bedeutet; sowie deren physiologisch verträgliche Salze;

wobei sich Aminosäurereste bzw. Aminosäureseitenketten von folgenden Aminosäuren ableiten können: Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Om, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

**2.** Heterocyclen gemäß Anspruch 1, dadurch gekennzeichnet, daß in der allgemeinen Formel I

Y - $(CH_2)_m$-CO-, wobei m für 1 oder 2 steht, oder -$CHR^s$-CO-, wobei $R^s$ für die Seitenkette der Aminosäuren Alanin, Valin, Phenylalanin, Tyrosin, Leucin, Isoleucin, Tryptophan, Lysin, Histidin, Asparagin, Glutamin oder

Phenylglycin steht, oder

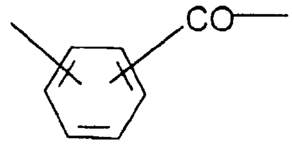

bedeutet;

r für 1 steht;

W $-COW^1$ bedeutet;

$Z^2$

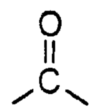

bedeutet;

$W^1$ Hydroxy, $(C_1-C_4)$-Alkoxy, insbesondere Methoxy, Ethoxy, 2-Propyloxy, Isobutyloxy oder tert.-Butyloxy, oder Benzyloxy bedeutet;

R Wasserstoff bedeutet;

$R^1$ $-(CH_2)_n-NH-X$, wobei n für eine ganze Zahl von 1 bis 5 steht, $-(CH_2)_p-C_6H_4-(CH_2)_q-NH-X$ oder $-(CH_2)_p-C_6H_4-(CH_2)_q-C(=NX)-NH_2$, wobei p und q unabhängig voneinander für 0 oder 1 stehen, bedeutet;

X Wasserstoff, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryl-$(C_1-C_6)$-alkoxycarbonyl oder einen Rest der Formel

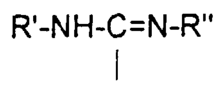

bedeutet, worin R' und R" unabhängig voneinander für Wasserstoff, Trifluorethyl, $(C_1-C_6)$-Alkylcarbonyl, $(C_1-C_6)$-Alkoxycarbonyl, $(C_1-C_{18})$- Alkylcarbonyloxy-$(C_1-C_6)$-alkoxycarbonyl oder $(C_6-C_{14})$-Aryl-$(C_1-C_6)$-alkoxycarbonyl stehen;

$R^2$ Wasserstoff bedeutet;

$R^3$ $-CO-NH-R^4$ bedeutet, wobei $-NH-R^4$ für den Rest einer α-Aminosäure, deren ω-Amino-$(C_2-C_8)$-alkylamid oder deren $(C_1-C_8)$-Alkyl- oder Benzylester steht, oder wobei $R^4$ Methyl bedeutet, das durch eine Aminosäureseitenkette und durch einen Rest aus der Reihe $-SO_2-OH$, $-SO_2-NHR^9$, Tetrazolyl substituiert ist.

**3.** Heterocyclen gemäß Anspruch 2, dadurch gekennzeichnet, daß $R^3$ $-CO-NH-R^4$ bedeutet, wobei $-NH-R^4$ für den Rest der α-Aminosäuren Valin, Lysin, Phenylalanin, Phenylglycin oder 4-Chlorphenylglycin, deren ω-Amino-$(C_2-C_8)$-alkylamide oder deren $(C_1-C_8)$-Alkyl- oder Benzylester steht.

**4.** Heterocyclen gemäß Anspruch 3, dadurch gekennzeichnet, daß der $(C_1-C_8)$-Alkylester der α-Aminosäuren der Methyl-, Ethyl-, Isopropyl-, Isobutyl- oder tert.-Butylester ist.

**5.** Verfahren zur Herstellung von Verbindungen der allgemeinen Formel I gemäß einem oder mehreren der Ansprüche 1 bis 4, dadurch gekennzeichnet, daß man eine Fragmentkondensation einer Verbindung der allgemeinen Formel III

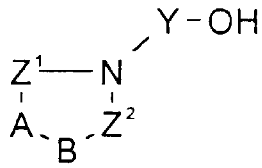

(III)

mit einer Verbindung der allgemeinen Formel IV ausführt,

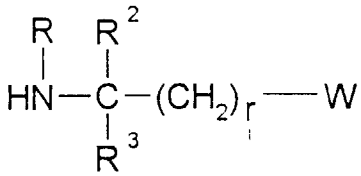

(IV)

wobei die Reste A, B, $Z^1$, $Z^2$, W, Y, R, $R^2$ und $R^3$ sowie r wie in den Ansprüchen 1 bis 4 angegeben definiert sind.

**6.** Verbindung der allgemeinen Formel I gemäß einem oder mehreren der Ansprüche 1 bis 4 und/oder ihre physiologisch verträglichen Salze zur Anwendung als Hemmstoffe der Thrombozytenaggregation, der Metastasierung von Karzinomzellen oder der Osteoclastenbindung an die Knochenoberfläche.

**7.** Pharmazeutisches Präparat, dadurch gekennzeichnet, daß es eine oder mehrere Verbindungen der allgemeinen Formel I gemäß einem oder mehreren der Ansprüche 1 bis 4 und/oder ein oder mehrere physiologisch verträgliche Salze davon als Wirkstoff zusammen mit pharmazeutisch annehmbaren Träger- und Zusatzstoffen und gegebenenfalls noch ein oder mehrere andere pharmakologische Wirkstoffe enthält.

**8.** Verfahren zur Herstellung eines pharmazeutischen Präparates, enthaltend eine oder mehrere Verbindungen der allgemeinen Formel I gemäß einem oder mehreren der Ansprüche 1 bis 4 und/oder ein oder mehrere physiologisch verträgliche Salze davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

**Claims**

**1.** A heterocycle of the formula I

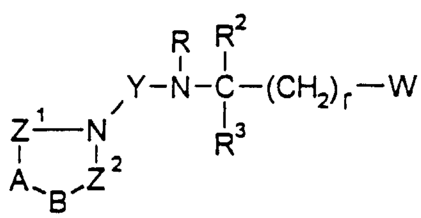

(I)

in which

$Z^1$ is

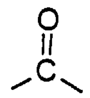 or 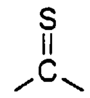 ;

$Z^2$ is

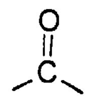 or 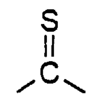

Y is $-(CH_2)_m-CO-$, where m is an integer from 1 to 4, or $-CHR^s-CO-$ or

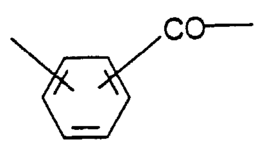 ;

r is an integer from 0 to 3;

A is $-CHR^1-$;

B is $-CH_2-$ or -O-;

W is $-COW^1$, tetrazolyl, $-SO_2-OH$ or $-SO_2-NHR^9$;

$W^1$ is hydroxyl, $(C_1-C_{28})$-alkoxy, $(C_6-C_{14})$-aryl-$(C_1-C_8)$-alkoxy, $(C_6-C_{14})$-aryloxy, amino or mono- or di-($(C_1-C_{18})$-alkyl)amino;

R is hydrogen or $(C_1-C_6)$-alkyl;

$R^1$ is

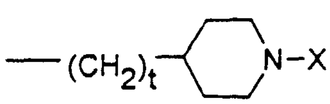

or $-(CH_2)_n-NH-X$ or $-(CH_2)_p-C_6H_4-(CH_2)_q-NH-X$ or $-(CH_2)_p-C(=NX)-NH_2$ or $-(CH_2)_p-C_6H_4-(CH_2)_q-C(=NX)-NH_2$, in which n is a number from 1 to 6, p and q independently of one another are a number from 0 to 3 and t is a number from 0 to 2;

X is hydrogen, $(C_1-C_6)$-alkyl, $(C_1-C_6)$-alkylcarbonyl, $(C_1-C_6)$-alkoxycarbonyl, $(C_1-C_{18})$-alkylcarbonyloxy-$(C_1-C_6)$-alkoxycarbonyl, $(C_6-C_{14})$-arylcarbonyl, $(C_6-C_{14})$-aryloxycarbonyl, $(C_6-C_{14})$-aryl-$(C_1-C_6)$-alkoxycarbonyl, cyano, hydroxyl, $(C_1-C_6)$-alkoxy or amino or a radical of the formula II

$$R'-NH-C(=N-R'')- \qquad (II)$$

where R' and R" independently of one another are hydrogen, $(C_1-C_6)$-alkyl, trifluoro-$(C_1-C_6)$-alkyl, $(C_1-C_6)$-alkoxycarbonyl, $(C_1-C_6)$-alkylcarbonyl, $(C_6-C_{14})$-arylcarbonyl, $(C_1-C_{18})$-alkylcarbonyloxy-$(C_1-C_6)$-alkoxycarbonyl, $(C_6-C_{14})$-aryloxycarbonyl, $(C_6-C_{14})$-aryl-$(C_1-C_6)$-alkoxycarbonyl, cyano, hydroxyl, $(C_1-C_6)$-alkoxy or amino;

$R^2$ is hydrogen, $(C_1-C_4)$-alkyl or phenyl, where the $(C_1-C_4)$-alkyl and the phenyl can be unsubstituted or mono- or polysubstituted by identical or different radicals from the group consisting of hydroxyl, amino, $(C_1-C_4)$-alkoxy, imidazolyl, indolyl, pyrrolidinyl, hydroxypyrrolidinyl, phenyl or halogen;

$R^3$ is hydrogen, $-COOR^4$, $-CO-N(CH_3)-R^4$ or $-CO-NH-R^4$;

$R^4$ is hydrogen or ($C_1$-$C_{28}$)-alkyl which can optionally be mono- or polysubstituted by identical or different radicals from the group consisting of hydroxyl, hydroxycarbonyl, aminocarbonyl, mono- or di-(($C_1$-$C_{18}$)-alkyl)aminocarbonyl, amino-($C_2$-$C_{18}$)-alkylaminocarbonyl, amino-($C_1$-$C_3$)-alkylphenyl-($C_1$-$C_3$) -alkylaminocarbonyl, ($C_1$-$C_{18}$)-alkylcarbonylamino-($C_1$-$C_3$)-alkylphenyl- ($C_1$-$C_3$)-alkylaminocarbonyl, ($C_1$-$C_{18}$)-alkylcarbonylamino-($C_2$-$C_{18}$)-alkylaminocarbonyl, ($C_6$-$C_{14}$)-aryl-($C_1$-$C_8$)-alkoxycarbonyl, amino, mercapto, ($C_1$-$C_{18}$)-alkoxy, ($C_1$-$C_{18}$)-alkoxycarbonyl, ($C_3$-$C_8$)-cycloalkyl, halogen, nitro, trifluoromethyl or by the radical $R^5$, where

$R^5$ is ($C_6$-$C_{14}$)-aryl, ($C_6$-$C_{14}$)-aryl-($C_1$-$C_8$)-alkyl, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, is a radical $R^6$ or is a radical $R^6$CO-, where the aryl radical and, independently thereof, the heterocyclic radical can be mono- or polysubstituted by identical or different radicals from the group consisting of ($C_1$-$C_{18}$)-alkyl, ($C_1$-$C_{18}$)-alkoxy, halogen, nitro, amino or trifluoromethyl;

$R^6$ is -$NR^7R^8$, -$OR^7$, -$SR^7$, -$SO_2$-OH, -$SO_2NHR^9$, tetrazolyl, an amino acid side chain, a natural or unnatural amino acid, imino acid, azaamino acid or dipeptide radical, and esters and amides thereof, where free functional groups can be protected by protective groups customary in peptide chemistry;

$R^7$ is hydrogen, ($C_1$-$C_{18}$)-alkyl, ($C_6$-$C_{14}$)-aryl-($C_1$-$C_8$)-alkyl, ($C_1$-$C_{18}$)-alkylcarbonyl, (01-018) -alkoxycarbonyl, ($C_6$-$C_{14}$)-arylcarbonyl, ($C_6$-$C_{14}$)-aryl($C_1$-$C_{18}$)-alkylcarbonyl or ($C_6$-$C_{14}$)-aryl-($C_1$-$C_{18}$)-alkyloxycarbonyl, where the alkyl groups can optionally be substituted by an amino group and/or where the aryl radicals can be mono- or polysubstituted, preferably monosubstituted, by identical or different radicals from the group consisting of ($C_1$-$C_8$)-alkyl, ($C_1$-$C_8$)-alkoxy, halogen, nitro, amino and trifluoromethyl, a natural or unnatural amino acid, imino acid, azaamino acid or a dipeptide radical;

$R^8$ is hydrogen, ($C_1$-$C_{18}$)-alkyl, ($C_6$-$C_{14}$)-aryl or ($C_6$-$C_{14}$)-aryl-($C_1$-$C_8$)-alkyl;

$R^9$ is hydrogen, aminocarbonyl, ($C_1$-$C_{18}$)-alkylaminocarbonyl, (C3-$C_8$)-cycloalkylaminocarbonyl, ($C_1$-$C_{18}$)-alkyl or ($C_3$-$C_8$)-cycloalkyl;

$R^s$ is an amino acid side chain;

or a physiologically tolerable salt thereof; where amino acid radicals or amino acid side chains can be derived from the following amino acids:

Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-diphenylaminoacetic acid, 2-(p-tolyl)-2-phenylaminoacetic acid, 2- (p-chlorophenyl)aminoacetic acid.

**2.** A heterocycle as claimed in claim 1, wherein, in the formula I,

Y is -$(CH_2)_m$-CO-, where m is 1 or 2, or -$CHR^s$-CO-, where $R^s$ is the side chain of the amino acids alanine, valine, phenylalanine, tyrosine, leucine, isoleucine, tryptophan, lysine, histidine, asparagine, glutamine or phenylglycine, or

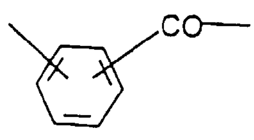

;

r is 1;

W is -$COW^1$;

$Z^2$ is

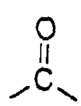

;

$W^1$ is hydroxyl, $(C_1-C_4)$-alkoxy, in particular methoxy, ethoxy, 2-propyloxy, isobutyloxy or tert-butyloxy, or benzyloxy;

R is hydrogen;

$R^1$ is $-(CH_2)_n$-NH-X, where n is an integer from 1 to 5, $-(CH_2)_p-C_6H_4-(CH_2)_q$-NH-X or $-(CH_2)_p-C_6H_4-(CH_2)_q$-C(=NX)-$NH_2$, where p and q independently of one another are 0 or 1;

X is hydrogen, $(C_1-C_6)$-alkoxycarbonyl, $(C_1-C_6)$-alkylcarbonyl, $(C_1-C_{18})$-alkylcarbonyloxy-$(C_1-C_6)$-alkoxycarbonyl, $(C_6-C_{14})$ -aryl- $(C_1-C_6)$ -alkoxycarbonyl or a radical of the formula

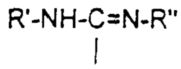

in which R' and R" independently of one another are hydrogen, trifluoroethyl, $(C_1-C_6)$-alkylcarbonyl, $(C_1-C_6)$-alkoxycarbonyl, $(C_1-C_{18})$-alkylcarbonyloxy-$(C_1-C_6)$-alkoxycarbonyl or $(C_6-C_{14})$-aryl-$(C_1-C_6)$-alkoxycarbonyl;

$R^2$ is hydrogen;

$R^3$ is -CO-NH-$R^4$, where -NH-$R^4$ is the radical of an α-amino acid, its ω-amino-$(C_2-C_8)$-alkylamide or its $(C_1-C_8)$-alkyl or benzyl ester, or where $R^4$ is methyl which is substituted by an amino acid side chain and by a radical from the group consisting of $-SO_2$-OH, $-SO_2$-NHR$^9$, tetrazolyl.

3. A heterocycle as claimed in claim 2, wherein $R^3$ is -CO-NH-$R^4$, where -NH-$R^4$ is the radical of the α-amino acids valine, lysine, phenylalanine, phenylglycine or 4-chlorophenylglycine, their ω-amino$(C_2-C_8)$-alkylamides or their $(C_1-C_8)$-alkyl or benzyl esters.

4. A heterocycle as claimed in claim 3, wherein the $(C_1-C_8)$-alkyl ester of the α-amino acids is the methyl, ethyl, isopropyl, isobutyl or tert-butyl ester.

5. A process for the preparation of compounds of the formula I as claimed in one or more of claims 1 to 4, which comprises carrying out a fragment condensation of a compound of the formula III

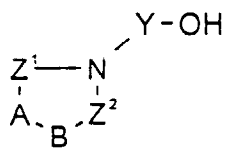

(III)

with a compound of the formula IV

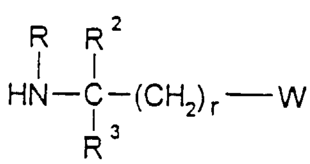

(IV)

where the radicals A, B, $Z^1$, $Z^2$, W, Y, R, $R^2$ and $R^3$ and also r are defined as indicated in claims 1 to 4.

6. A compound of the formula I as claimed in one or more of claims 1 to 4 and/or its physiologically tolerable salts for use as an inhibitor of platelet aggregation, metastasis of carcinoma cells or osteoclast binding to the bone surface.

7. A pharmaceutical preparation which contains one or more compounds of the formula I as claimed in one or more of claims 1 to 4 and/or one or more physiologically tolerable salts thereof as active compound together with pharmaceutically acceptable excipients and additives and, if appropriate, additionally one or more other pharmacological active compounds.

8. A process for the production of a pharmaceutical preparation comprising one or more compounds of the formula I as claimed in one or more of claims 1 to 4 and/or one or more physiologically tolerable salts thereof, which comprises bringing these into a suitable administration form together with pharmaceutically acceptable excipients and additives and, if appropriate, additionally one or more other pharmacological active compounds.

## Revendications

1. Hétérocycles de formule générale I

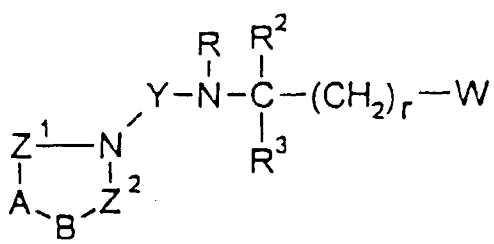

(I)

dans laquelle

$Z^1$ représente

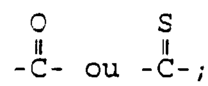

$Z^2$ représente

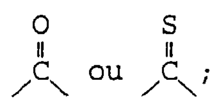

Y représente $-(CH_2)_m-CO-$, m représentant un nombre entier allant de 1 à 4, ou $-CHR^s-CO-$ ou

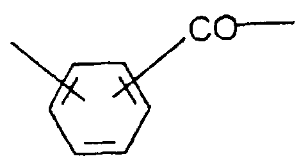

r représente un nombre entier allant de 0 à 3;
A représente $-CHR^1-$;
B représente $-CH_2-$ ou -O-;
W représente un groupe $-COW^1$, tétrazolyle, $-SO_2-OH$ ou $-SO_2-NHR^9$;
$W^1$ représente un groupe hydroxyle, alcoxy en $C_1$-$C_{28}$, aryl-($C_6$-$C_{14}$)alcoxy($C_1$-$C_8$), aryloxy en $C_6$-$C_{14}$, amino ou mono- ou dialkyl($C_1$-$C_{18}$)amino;
R représente un atome d'hydrogène ou un groupe alkyle en $C_1$-$C_6$;
$R^1$ représente un groupe

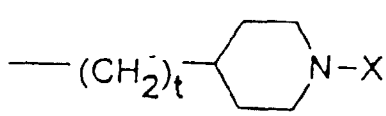

ou $(CH_2)_n$-NH-X ou $-(CH_2)_p-C_6H_4-(CH_2)_q$-NH-X ou $-(CH_2)_p$-C(=NX)-$NH_2$ ou $-(CH_2)_p-C_6H_4-(CH_2)_q$-C(=NX)-$NH_2$, groupes dans lesquels n représente un nombre allant de 1 à 6, p et q représentent, indépendamment l'un de l'autre, un nombre allant de 0 à 3, et t représente un nombre allant de 0 à 2;

X représente un atome d'hydrogène ou un groupe alkyle en $C_1$-$C_6$, alkyl($C_1$-$C_6$)carbonyle, alcoxy($C_1$-$C_6$)carbonyle, alkyl($C_1$-$C_{18}$)carbonyloxy-alcoxy($C_1$-$C_6$)carbonyle, aryl-($C_6$-$C_{14}$)carbonyle, aryloxy($C_6$-$C_{14}$)carbonyle, aryl-($C_6$-$C_{14}$)alcoxy($C_1$-$C_6$)carbonyle, cyano, hydroxyle, alcoxy en $C_1$-$C_6$ ou amino, ou un radical de formule II

R'-NH-C(=N-R")- (II)

R' et R" représentant, indépendamment l'un de l'autre, un atome d'hydrogène ou un groupe alkyle en $C_1$-$C_6$, trifluoroalkyle en $C_1$-$C_6$, alcoxy($C_1$-$C_6$)carbonyle, alkyl-($C_1$-$C_6$)carbonyle, aryl ($C_6$-$C_{14}$) carbonyle, alkyl($C_1$-$C_{18}$)-carbonyloxy-alcoxy($C_1$-$C_6$)carbonyle, aryloxy($C_6$-$C_{14}$)-carbonyle, aryi($C_6$-$C_{14}$)alcoxy($C_1$-$C_6$)carbonyle, cyano, hydroxyle, alcoxy en $C_1$-$C_6$ ou amine;

$R^2$ représente un atome d'hydrogène ou un groupe alkyle en $C_1$-$C_4$ ou phényle, le groupe alkyle en $C_1$-$C_4$ et le groupe phényle pouvant être non substitués ou une ou plusieurs fois substitués par des radicaux, identiques ou différents, choisis parmi des atomes d'halogène et des groupes hydroxyle, amino, alcoxy en $C_1$-$C_4$, imidazolyle, indolyle, pyrrolidinyle, hydroxypyrrolidinyle et phényle;

$R^3$ représente un atome d'hydrogène ou un groupe $COOR^4$, -CO-N($CH_3$)-$R^4$ ou -CO-NH-$R^4$;

$R^4$ représente un atome d'hydrogène ou un groupe alkyle en $C_1$-$C_{28}$, qui peut éventuellement être une ou plusieurs fois substitué par des radicaux identiques ou différents choisis parmi des groupes hydroxyle, hydroxycarbonyle, aminocarbonyle, mono- ou dialkyl($C_1$-$C_{18}$)-aminocarbonyle, aminoalkyl($C_2$-$C_{18}$)aminocarbonyle, aminoalkyl($C_1$-$C_3$)phényl-alkyl($C_1$-$C_3$)aminocarbonyle, alkyl($C_1$-$C_{18}$)carbonylamino-alkyl($C_1$-$C_3$)phénylalkyl-($C_1$-$C_3$)aminocarbonyle, alkyl($C_1$-$C_{18}$)carbonylaminoalkyl($C_2$-$C_{18}$)aminocarbonyle, aryl($C_6$-$C_{14}$)alcoxy-($C_1$-$C_8$)carbonyle, amino, mercapto, alcoxy en $C_1$-$C_{18}$, alcoxy($C_1$-$C_{18}$)carbonyle, cycloalkyle en $C_3$-$C_8$, des atomes d'halogène, le groupe nitro, le groupe trifluorométhyle, ou par le radical $R^5$,

$R^5$ représentant un groupe aryle en $C_6$-$C_{14}$, aryl-($C_6$-$C_{14}$)alkyle($C_1$-$C_8$), un cycle hétérocyclique mono-ou bicyclique à 5-12 chaînons, qui peut être aromatique, partiellement hydrogéné ou totalement hydrogéné, et qui peut comporter 1, 2 ou 3 hétéroatomes identiques ou différents choisis parmi les atomes d'azote, d'oxygène et de soufre, un radical $R^6$ ou un radical $R^6$CO-, le radical aryle et, indépendamment de celui-ci, le radical hétérocyclique pouvant porter un ou plusieurs substituants, identiques ou différents, choisis parmi des atomes d'halogène et des groupes alkyle en $C_1$-$C_{18}$, alcoxy en $C_1$-$C_{18}$, nitro, amino ou trifluorométhyle;

$R^6$ représentant un groupe $-NR^7R^8$, $-OR^7$, $-SR^7$, $-SO_2$-OH, $-SO_2NHR^9$, tétrazolyle, une chaîne latérale d'aminoacide, un reste d'aminoacide, d'iminoacide, d'azaaminoacide, naturel ou non naturel, ou un reste de dipeptide, ainsi que de leurs esters et amides, des groupes fonctionnels libres pouvant être protégés par des groupes protecteurs usuels dans la chimie des peptides;

$R^7$ représentant un atome d'hydrogène ou un groupe alkyle en $C_1$-$C_{18}$, aryl($C_6$-$C_{14}$)-alkyle($C_1$-$C_8$), alkyl-($C_1$-$C_{18}$)carbonyle, alcoxy($C_1$-$C_{18}$)carbonyle, aryl-($C_6$-$C_{14}$)carbonyle, aryl($C_6$-$C_{14}$)-alkyl($C_1$-$C_8$)-carbonyle ou aryl ($C_6$-$C_{14}$)-alkyloxy($C_1$-$C_{18}$)carbonyle, les groupes alkyle pouvant éventuellement être substitués par un groupe amino et/ou les radicaux aryle pouvant être substitués une ou plusieurs fois, de préférence une fois, par des radicaux, identiques ou différents, choisis dans l'ensemble constitué par des atomes d'halogène et des groupes alkyle en $C_1$-$C_8$, alcoxy en $C_1$-$C_8$, nitro, amino et trifluorométhyle, ou représentant un reste d'aminoacide, d'iminoacide, d'aza-aminoacide, naturel ou non naturel, ou de dipeptide;

$R^8$ représentant un atome d'hydrogène ou un groupe alkyle en $C_1$-$C_{18}$, aryle en $C_6$-$C_{14}$ ou aryl($C_6$-$C_{14}$)-alkyle ($C_1$-$C_8$);

$R^9$ représentant un atome d'hydrogène ou un groupe aminocarbonyle, alkyl($C_1$-$C_{18}$)aminocarbonyle, cycloalkyl ($C_3$-$C_8$)aminocarbonyle, alkyle en $C_1$-$C_{18}$ ou cycloalkyle en $C_3$-$C_8$;

$R^s$ représente une chaîne latérale d'aminoacide;

ainsi que leurs sels physiologiquement acceptables;
les restes d'aminoacides ou les chaînes latérales d'aminoacides pouvant provenir des aminoacides suivants: Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, l'acide 2,2-diphénylaminoacétique, l'acide 2-(p-tolyl)-2-phénylaminoacétique, l'acide 2-(p-chlorophényl)aminoacétique.

**2.** Hétérocycles selon la revendication 1, caractérisés en ce que, dans la formule générale I

Y représente $-(CH_2)_m-CO-$, m représentant 1 ou 2, ou $-CHR^sCO-$, $R^s$ représentant la chaîne latérale de l'aminoacide alanine, valine, phénylalanine, tyrosine, leucine, isoleucine, tryptophane, lysine, histidine, asparagine, glutamine ou phénylglycine, ou

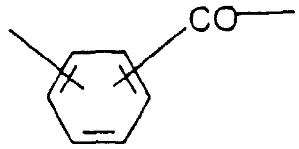

r représente 1;
W représente $-COW^1$;
$Z^2$ représente

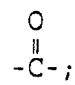

$W^1$ représente un groupe hydroxyle, alcoxy en $C_1$-$C_4$, en particulier méthoxy, éthoxy, 2-propyloxy, isobutyloxy ou tert-butyloxy, ou benzyloxy;
R représente un atome d'hydrogène;
$R^1$ représente un groupe $-(CH_2)_n-NH-X$, n représentant un nombre entier allant de 1 à 5, $-(CH_2)_p-C_6H_4-(CH_2)_q-NH-X$ ou $(CH_2)_p-C_6H_4-(CH_2)_q-C(=NX)-NH_2$, p et q représentant, indépendamment l'un de l'autre, 0 ou 1;
X représente un atome d'hydrogène ou un groupe alcoxy-($C_1$-$C_6$)carbonyle, alkyl($C_1$-$C_6$)carbonyle, alkyl($C_1$-$C_{18}$)-carbonyloxy-alcoxy($C_1$-$C_6$) carbonyle, aryl($C_6$-$C_{14}$)alcoxy-($C_1$-$C_6$)carbonyle ou un radical de formule

$$R'-NH-C(=N-R'')-$$

dans laquelle R' et R" représentent, indépendamment l'un de l'autre, un atome d'hydrogène ou un groupe trifluoroéthyle, alkyl ($C_1$-$C_6$)carbonyle, alcoxy($C_1$-$C_6$)-carbonyle, alkyl($C_1$-$C_{18}$) carbonyloxy-alcoxy($C_1$-$C_6$)-carbonyle ou aryl($C_6$-$C_{14}$)alcoxy($C_1$-$C_6$) carbonyle;
$R^2$ représente un atome d'hydrogène;
$R^3$ représente un groupe $-CO-NH-R^4$, $-NH-R^4$ représentant le reste d'un α-aminoacide, d'un ω-amino-alkyl($C_2$-$C_8$)amide ou d'un ester alkylique en $C_1$-$C_8$ ou benzylique de celui-ci, ou $R^4$ représentant un groupe méthyle qui est substitué par une chaîne latérale d'aminoacide et par un radical choisi dans l'ensemble constitué par les groupes $-SO_2-OH$, $-SO_2-NHR^9$ et tétrazolyle.

**3.** Hétérocycles selon la revendication 2, caractérisés en ce que $R^3$ représente un groupe $-CO-NH-R^4$, $-NH-R^4$ représentant le reste de l'α-aminoacide valine, lysine, phénylalanine, phénylglycine ou 4-chlorophénylglycine, d'un ω-amino-alkyl($C_2$-$C_8$)amide d'un tel aminoacide ou d'un ester alkylique en $C_1$-$C_8$ ou benzylique d'un tel aminoacide.

**4.** Hétérocycles selon la revendication 3, caractérisés en ce que l'ester alkylique en $C_1$-$C_8$ des α-aminoacides est l'ester méthylique, éthylique, isopropylique, isobutylique ou tert-butylique.

**5.** Procédé pour la préparation des composés de formule générale I selon une ou plusieurs des revendications 1 à 4, caractérisé en ce que l'on effectue une condensation de fragments, en condensant un composé de formule générale III

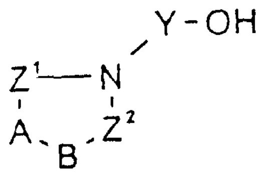

(III)

avec un composé de formule générale IV

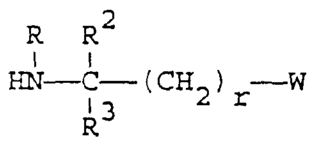

(IV)

les radicaux $Z^1$, $Z^2$, A, B, R, $R^2$, $R^3$, Y et W ainsi que r étant définis comme indiqué dans les revendication 1 à 4.

**6.** Composé de formule générale I selon une ou plusieurs des revendication 1 à 4, et/ou leurs sels physiologiquement acceptables, pour utilisation en tant qu'inhibiteurs de l'agrégation des thrombocytes, de la dissémination de métastases de cellules de carcinomes ou de la fixation d'ostéoclastes à la surface des os.

**7.** Composition pharmaceutique, caractérisée en ce qu'elle contient, en tant que substance active, un ou plusieurs des composés de formule générale I selon une ou plusieurs des revendications 1 à 4, et/ou un ou plusieurs sels physiologiquement acceptables de celui-ci(ceux-ci), conjointement avec des additifs et véhicules pharmaceutiquement acceptables et éventuellement encore une ou plusieurs autres substances pharmacologiquement actives.

**8.** Procédé pour la préparation d'une composition pharmaceutique, contenant un ou plusieurs des composés de formule générale I selon une ou plusieurs des revendications 1 à 4, et/ou un ou plusieurs sels physiologiquement acceptables de ceux-ci, caractérisé en ce qu'on les met sous une forme d'administration appropriée, conjointement avec des additifs et véhicules pharmaceutiquement acceptables et éventuellement encore une ou plusieurs autres substances pharmacologiquement actives.